# EUROPEAN PATENT SPECIFICATION

(11) **EP 3 919 050 B1**
(45) Date of publication and mention of the grant of the patent: **06.05.2026**
(21) Application number: 20178426.1
(22) Date of filing: 05.06.2020
(51) Int. Cl.: A61K 31/015, A61K 36/185, A61K 36/752, A61P 3/04, A23L 33/00, A23L 33/105, A23L 33/21, A23L 33/22

(54) **NUTRACEUTIC COMPOSITION CONTAINING LIMONENE AND A DRY EXTRACT OF COCOA FIBRE AND ITS USE FOR THE TREATMENT OF OBESITY**
NAHRUNGSMITTELZUSAMMENSETZUNG ENTHALTEND LIMONEN UND EIN TROCKENES KAKAOFASEREXTRAKT UND DESSEN VERWENDUNG ZUR BEHANDLUNG IN FETTLEIBIGKEIT
COMPOSITION NUTRACEUTIQUE COMPRENANT LIMONENE ET UN EXTRAIT SEC DES FIBRES DE CACAO ET SON UTILISATION POUR LE TRAITEMENT DE L' OBÉSITÉ

(43) Date of publication of application: 08.12.2021
(73) Proprietor: Targeting Gut Disease S.r.l., 40127 Bologna, BO (IT)
(72) Inventor: Valerii, Maria Chiara, 40127 Bologna BO (IT)
(74) Representative: Longoni, Alessandra

(56) References cited:
- US-A1- 2003 206 981
- US-A1- 2005 277 657
- US-B2- 8 709 503
- GALVAGNI ELISIANE ET AL: "Detection of Volatiles in Dark Chocolate Flavored with Orange Essential Oil by Electronic Nose", FOOD ANALYTICAL METHODS, SPRINGER NEW YORK LLC, US, vol. 13, no. 7, 8 May 2020 (2020-05-08), pages 1421 - 1432, XP037181948, ISSN: 1936-9751, [retrieved on 20200508], DOI: 10.1007/S12161-020-01763-X
- JING LI ET AL: "Preventive and ameliorating effects of citrusd-limonene on dyslipidemia and hyperglycemia in mice with high-fat diet-induced obesity", EUROPEAN JOURNAL OF PHARMACOLOGY, ELSEVIER SCIENCE, NL, vol. 715, no. 1, 6 July 2013 (2013-07-06), pages 46 - 55, XP028705003, ISSN: 0014-2999, DOI: 10.1016/J.EJPHAR.2013.06.022
- VALERII MARIA CHIARA ET AL: "Effect of a Fiber D-Limonene-Enriched Food Supplement on Intestinal Microbiota and Metabolic Parameters of Mice on a High-Fat Diet", PHARMACEUTICS, vol. 13, no. 11, 21 October 2021 (2021-10-21), Switzerland, pages 1753, XP093231251, ISSN: 1999-4923, DOI: 10.3390/pharmaceutics13111753

## Description

The present invention relates to a nutraceutical composition comprising essential oils and cocoa fibre, more in particular to a nutraceutical composition comprising natural or synthetic limonene or an essential oil having a content of limonene of at least 30% by weight and cocoa fibre for human or veterinary therapeutic use in the treatment of obesity.

Essential oils and their active ingredients have particularly attractive biological activities especially in the case of oral consumption.

Conveniently dosed, they can be used for their slimming, germicidal, fungicidal, antibacterial, anti-inflammatory, anti-parasite and antibiotic activities (Li et al, 2013, EJP, 715; 46-55; US 2005/277657; Thapa et al., 2012, Microbiology, 158 (11); 2870-2877). However, these substances present enormous problems in therapeutic applications due both to their aggressiveness with regard to the oral, oesophagus, gastric and intestinal mucus and their toxic nature.

Even using correct therapeutic doses, extracts of vegetable origin that contain essential oils and/or their components (including synthetic analogues) may cause inflammations, irritations, erosions or other damages to the mucus with which they come into contact, such as for example in the mouth.

Moreover, if for example, a therapeutic action of essential oils is sought, it is very difficult to carry the corresponding active ingredients to the streches of interest without damaging, even in a significant manner, the intestinal tracts passed through before reaching the area of interest. Even the fast absorption of essential oils, especially in the upper intestinal tract (called small intestine), can prevent them from effectively reaching the lower tracts, such as colon for example, where they would exert their therapeutic action topically. Moreover, in some diseases, it would be recommended to have both a topical and systemic effect of the active components that should act in the digestive tract and in the bloodstream on circulating molecules.

The combination of the topical and systemic effect is essential in the treatment of obesity with natural or synthetic limonene or with essential oils containing limonene, in particular with orange essential oil.

Orange essential oils and their components, in particular limonene, are known to have an anti-obesity effect (Dhu Li et al., Bioscience, Biotechnology, and Biochemistry, vol. 83(5), 2019, 923-932).

The patent application EP 3 097 921 filed in the name of the Applicant discloses a composition comprising essential oils and a dry plant extract, root, sprout or fruit extract that allows to overcome the above-mentioned drawbacks and to administer essential oils or components thereof so that they exert their topical action in the intestine without damaging the mucosa and organs of the digestive tract.

Although particularly advantageous and innovative for administering essential oils or components thereof, the composition disclosed in EP 3 097 921 comprising dry extract, in particular dry extract of glucomannan, ginger root, turmeric root, boswellia, cabbage and fabaceae seeds, does not guarantee also an effective systemic effect in case of administration of essential oils or components thereof for treating obesity. The Application has now found that the dual local and systemic effect is obtained by using cocoa fibre instead of dry plant extracts, root, sprout or fruit extracts disclosed in EP 3 097 921.

The object of the present invention is therefore a nutraceutical composition comprising natural or synthetic limonene or an essential oil having a content of limonene of at least 30% by weight and cocoa fibre. in a quantity comprised between 50% and 95% by weight.

The composition according to this invention allows to cancel or significantly reduce the negative effects of the essential oil or components thereof on mucus, to partially release the active ingredient in the first part of the digestive tract so that it is absorbed and that another part of the active ingredient can reach the other parts of the digestive tract and locally exert its action.

The composition according to this invention, thanks to its peculiar dual local and systemic effect due to the use of cocoa fibre, is particularly suitable for the treatment of obesity.

In the composition according to this invention, a preferred essential oil is a citrus essential oil, in particular orange essential oil.

Non-limiting esamples of essential oils having a content of limonene of at least 30% by weight that can be used according to the present invention are the products marketed as "O.E. Arancio Sicilia", O.E. Arancio Brasile" and Natural Terpenes 100% from Industrie Chimiche Muller & Koster SpA.

According to a particularly preferred embodiment, the composition of the present invention comprises natural or synthetic limonene.

In the present description, the essential oil and the limonene are generically indicated as active ingredient.

Preferably, the active ingredient is present in the composition in a quantity comprised between 5% and 50% by weight, more preferably in a quantity comprised between 10% and 25% by weight.

The composition according to this invention is characterized by the fact that the active ingredient is mixed with cocoa fibre.

In the present description, the term "cocoa fibre" is used to indicate cocoa fibre powder or dry cocoa extract or cocoa bean powder or powder obtained from cocoa bean pericarp. The use of the term "cocoa fibre" also indicates that the main component is natural vegetable fibre.

The cocoa fibre used in the composition of the present invention is available on the market and is generally referred to as "cacao fibra polv." or "Theobroma cacao, Shell".

The cocoa fibre is present in the composition of the invention in a quantity comprised between 50% and 95% by weight, more preferably in a quantity comprised between 75% and 90% by weight.

Preferably, the natural fibre has a grain size comprised in a range of from -1 to +8 of the Krumbein (φ) phi scale (1934).

The compositions of the invention are prepared according to a similar process to that disclosed in the already-mentioned patent application EP 3 097 921 in the name of the Applicant.

Said process provides for mixing the active ingredient with cocoa fibre under continuous stirring in order to form solid spherical lumps of variable dimensions.

Said lumps are then subjected to a breaking step by mechanical action, followed preferably by a mixing step until complete dissolution of the lumps.

Advantageously, the composition according to the invention can be encapsulated in normal gelatine capsules, designed to contain liquid, solid and/or powder substances. In this way, the compositions of the invention allow the encapsulation of the active ingredient, which is normally volatile, in normal gelatine capsules for oral administration.

The compositions of the present invention are useful in the treatment of obesity for human or veterinary use.

A further object of the present invention is therefore the use of a nutraceutical composition comprising natural or synthetic limonene or an essential oil having a content of limonene of at least 30% by weight and cocoa fibre in a quantity comprised between 50% and 95% by weight for human or veterinary therapeutic use in the treatment of obesity.

In one embodiment, the composition for use in the treatment of obesity comprises also at least one pharmaceutically acceptable carrier that allows its formulation in conventional dosage forms, such as for example tablets, granules, capsules or suspensions for oral administration.

The preparation of these dosage forms and the selection of the pharmaceutically acceptable carriers are comprised within the knowledge of the skilled person.

The dose of the compositions according to the invention for human or veterinary use is preferably comprised between 5 mg and 10 g/day of composition, for example on the basis of the species, means of administration, age and weight of the subject.

By way of example and without limiting the scope of the invention, preferred embodiments of the composition according to the invention are described below.

### Example 1

Composition comprising:
- natural or synthetic limonene;
- cocoa fibre powder or dry cocoa extract or cocoa bean powder or powder obtained from cocoa bean pericarp.
   60 g of cocoa fibre are added to 10 g of natural or synthetic limonene.

After the addition of cocoa fibre to the natural or synthetic limonene, under continuous stirring, solid spherical lumps of variable dimensions are formed.

The lumps are broken by mechanical action (using for example a vibromixer or similar instrument designed to break the lumps) and the mixture is mixed until complete dissolution of the aggregates.

With reference to the above-mentioned examples, some results are reported below of animal and/or clinical trials supporting the therapeutic use.

Demonstration of the slimming effect according to example 1 in the treatment of obesity.

The composition of example 1 has been administered to a group of mice with obesity induced by a high lipid diet, with a dose of 60 mg/day for a cycle with a duration of 6 weeks.

The effectiveness of the treatment was demonstrated by monitoring the weight of the animals, which were well fed with a weight-gaining diet with a high lipid content. After 6 weeks of treatment, the group of animals treated had an average weight reduced by 12% compared with the group of control animals. The animals treated also showed lower blood sugar and triglyceride levels compared with the control group.

Demonstration of the slimming effect of limonene in obese mice.

Animal feed having a concentration of 0.5% in limonene corresponding to a daily dose of 0.6 g/kg of weight in humans. After 12 weeks of high lipid diet that caused obesity, mice were administered ad libitum, animal feed containing 0.5% of limonene for 2 weeks. The efficacy of the treatment was demonstrated by decreasing the size of both white and brown adipocytes, by reducing the serum triglycerides up to 47% and by preventing the accumulation of lipids in the cells.

The present invention therefore relates to a new mixing of natural or synthetic limonene or of essential oil having a content of limonene of at least 30% by weight, as active ingredient, with cocoa fibre used for the treatment of obesity in human or animals.

The composition is a damp powder (hygroscopic) which releases the active component in the area of interest. The specific cocoa fibre composition allows a partial and gradual release of limonene in the small intestine during transit where it is absorbed and exerts its important action on lipid and glycemic profile in obese individuals. In the large intestine, the oily remaining component, harnessed in fibre, is released when the fibre is degraded by the intestinal bacterial component.

At this moment, the remaining part of the active ingredient modulates the subject metabolism by acting on the intestinal microbiota.

Such a dual effect is obtained by using cocoa fibre but not with other vegetable fibres which convey the active ingredient almost exclusively through the colon but do not allow it to be absorbed.

The fibre also prevents the evaporation of the essential oil component and therefore allows it to be encapsulated in wrappers of any type.

The compositions according to the present invention are particularly advantageous since the obtained mixture is stable and the active component of the mixture (essential oils, components thereof or vegetable origin extracts which they contain, including synthetic analogues) is absorbed on a matrix which eliminates the majority of, or completely, the negative effects on the mucus, allowing a slow and controlled release, in particular along the whole intestinal tract.

The particular cocoa fibre composition allows to release the active partially in the small intestine where it is absorbed and can act systemically, partially in the colon where it can act topically. This characteristic is the basis for the efficacy of the composition.

From a biological point of view, the mixture according to the invention allows the active ingredient to be taken free of the side effects which there would be without mixing. It also allows a slower release of the active ingredient (retard effect), which while starting in the stomach, continues in the small and large intestine where the fibre is demolished by the intestinal microbiota (bacterial flora) and the active ingredient may perform its slimming effect along with its germicidal, fungicidal, antibacterial, anti-inflammatory, anti-parasite and antibiotic actions.

## Claims

1. A nutraceutical composition comprising natural or synthetic limonene or an essential oil having a content of limonene of at least 30% by weight, as active ingredient, and cocoa fibre in a quantity comprised between 50% and 95% by weight.

2. Composition according to claim 1 wherein the essential oil is orange essential oil.

3. Composition according to any one of the preceding claims wherein the cocoa fibre is cocoa fibre powder or dry cocoa extract or cocoa bean powder or powder obtained from cocoa bean pericarp.

4. Composition according to any one of the preceding claims wherein the limonene or the essential oil are present in a quantity comprised between 5% and 50% by weight, preferably in a quantity comprised between 10% and 25% by weight.

5. Composition according to any one of the preceding claims wherein the cocoa fibre is present in a quantity comprised between 75% and 90% by weight.

6. Composition according to claim 5 wherein the fibre has a grain size comprised in a range of from -1 to +8 of the Krumbein phi scale.

7. Process for the preparation of the composition according to any one of the preceding claims comprising mixing limonene or essential oil with cocoa fibre under continuous stirring in order to form solid spherical lumps of variable dimensions, followed by a breaking step by mechanical action, followed preferably by a mixing step until complete dissolution of the lumps.

8. Composition according to any one of claims 1-6 for human or veterinary therapeutic use in the treatment of obesity.

## Patentansprüche

1. Nutrazeutische Zusammensetzung, umfassend natürliches oder synthetisches Limonen oder ein ätherisches Öl mit einem Gehalt an Limonen von mindestens 30 Gew.-% als Wirkstoff und Kakaofaser in einer Menge umfassend zwischen 50 und 95 Gew.-%.

2. Zusammensetzung nach Anspruch 1, wobei das ätherische Öl ätherisches Orangenöl ist.

3. Zusammensetzung nach einem der vorhergehenden Ansprüche, wobei die Kakaofaser Kakaofaserpulver oder Kakaotrockenextrakt oder Kakaobohnenpulver oder Pulver, das aus Kakaobohnenperikarp erhalten wird, ist.

4. Zusammensetzung nach einem der vorhergehenden Ansprüche, wobei das Limonen oder das ätherische Öl in einer Menge umfassend zwischen 5 und 50 Gew.-%, vorzugsweise in einer Menge umfassend zwischen 10 und 25 Gew.-% vorhanden sind.

5. Zusammensetzung nach einem der vorhergehenden Ansprüche, wobei die Kakaofaser in einer Menge umfassend zwischen 75 und 90 Gew.-% vorhanden ist.

6. Zusammensetzung nach Anspruch 5, wobei die Faser eine Korngröße umfassend in einem Bereich von -1 bis +8 der Krumbein-Phi-Skala aufweist.

7. Verfahren zur Herstellung der Zusammensetzung nach einem der vorhergehenden Ansprüche, umfassend Mischen von Limonen oder ätherischem Öl mit Kakaofaser unter kontinuierlichem Rühren, um feste kugelförmige Klumpen mit variablen Abmessungen auszubilden, gefolgt von einem Schritt eines Brechens durch mechanische Einwirkung, vorzugsweise gefolgt von einem Schritt eines Mischens bis zur vollständigen Auflösung der Klumpen.

8. Zusammensetzung nach einem der Ansprüche 1-6 zur human- oder veterinärtherapeutischen Verwendung bei der Behandlung von Fettleibigkeit.

## Revendications

1. Composition nutraceutique comprenant du limonène naturel ou synthétique ou une huile essentielle présentant une teneur en limonène d'au moins 30 % en poids, en tant que principe actif, et de la fibre de cacao en une quantité comprise entre 50 % et 95 % en poids.

2. Composition selon la revendication 1, dans laquelle l'huile essentielle est une huile essentielle d'orange.

3. Composition selon l'une quelconque des revendications précédentes, dans laquelle la fibre de cacao est une poudre de fibre de cacao ou un extrait sec de cacao ou une poudre de fève de cacao ou une poudre obtenue à partir de péricarpe de fève de cacao.

4. Composition selon l'une quelconque des revendications précédentes, dans laquelle le limonène ou l'huile essentielle sont présents en une quantité comprise entre 5 % et 50 % en poids, de préférence en une quantité comprise entre 10 % et 25 % en poids.

5. Composition selon l'une quelconque des revendications précédentes, dans laquelle la fibre de cacao est présente en une quantité comprise entre 75 % et 90 % en poids.

6. Composition selon la revendication 5, dans laquelle la fibre présente une taille de grain comprise dans une plage de -1 à +8 sur l'échelle de Krumbein phi.

7. Processus permettant la préparation de la composition selon l'une quelconque des revendications précédentes comprenant le mélange de limonène ou d'huile essentielle avec de la fibre de cacao sous agitation continue afin de former des grumeaux sphériques solides de dimensions variables, suivi d'une étape de rupture par action mécanique, suivie de préférence d'une étape de mélange jusqu'à dissolution complète des grumeaux.

8. Composition selon l'une quelconque des revendications 1 à 6, destinée à un usage thérapeutique humain ou vétérinaire dans le traitement de l'obésité.
